# EUROPEAN PATENT SPECIFICATION

(11) **EP 1 649 003 B1**
(45) Date of publication and mention of the grant of the patent: **19.01.2011**
(21) Application number: 04777626.5
(22) Date of filing: 06.07.2004
(51) Int. Cl.: C12N 5/071

(54) **METHODS OF PRODUCING DIFFERENTIATED HEMATOPOIETIC CELLS FOR TREATMENT OF CYTOPENIA**
VERFAHREN ZUR HERSTELLUNG DIFFERENZIERTER HÄMATOPOETISCHER ZELLEN ZUR BEHANDLUNG VON ZYTOPENIE
PROCEDES DE PRODUCTION DE CELLULES HEMATOPOIETIQUES DIFFERENCIEES POUR LE TRAITEMENT DE LA CYTOPENIE

(30) Priority: 06.07.2003 US 485509 P; 07.07.2003 US 485607 P
(43) Date of publication of application: 26.04.2006
(73) Proprietor: Roger Williams Medical Center, Providence, RI 02908 (US)
(72) Inventor: QUESENBERRY, Peter, J., Providence, RI 02906 (US)
(74) Representative: Hallybone, Huw George
(86) International application number: PCT/US2004/021637
(87) International publication number: WO 2005/003321

(56) References cited:
- WO-A-99/40180
- WO-A-2004/056397
- QUESENBERRY PETER J ET AL: "The chiaroscuro stem cell: a unified stem cell theory." BLOOD. 15 DEC 2002, vol. 100, no. 13, 15 December 2002 (2002-12-15), pages 4266-4271, XP002398506 ISSN: 0006-4971
- HAGIHARA M ET AL: 'Extensive and long-term ex vivo production of dendritic cells from CD34 positive umbilical cord blood or bone marrow cells by novel culture system using mouse stroma.' J IMMUNOL METHODS. vol. 253, no. 1-2, July 2001, pages 45 - 55, XP004241359
- MILHEM MM ET AL: 'Reprogramming of primitive hematopoietic cells in vitro.' BLOOD. vol. 100, no. 11, November 2002, page 290A, XP008062698

## Description

### FIELD OF THE INVENTION

This application claims the benefit of U.S. Provisional Application Serial No. 60/485,509, filed July 6, 2003, and U.S. Provisional Application Serial No. 60/485,607, filed July 7, 2003.

### FIELD OF THE INVENTION

The present invention relates to in vitro methods for the production of differentiated hematopoietic cells by culturing synchronized bone marrow stem cells in the presence of cytokines at specific phases of the cell cycle. In one aspect, the invention provides differentiated hematopoietic cells, including megakaryocytes, platelets, and granulocytes. In another aspect, the invention provides methods of treating cytopenic states using the differentiated hematopoietic cells. The invention has a wide spectrum of useful applications including treatment of cytopenic states associated with cancer chemotherapy/chemoradiotherapy, bone marrow transplantation, and stem cell transplantation.

### BACKGROUND OF THE INVENTION

In general, models of stem cell regulation have been hierarchical (Quesenberry, P.J. Hematopoiesis and hematopoietic growth factors. In: Goldman, L. and Bennett, J.C. eds. Cecil Textbook of Medicine. 21st ed. Philadelphia, PA: WB Saunders; 1999:834-840; Quesenberry, P.J. and Golvin, G.A. Hematopoietic stem cells, progenitor cells and cytokines. In: Beutler, E. et al. eds. Williams Hematology. 6th ed. New York, NY: McGraw-Hill; 2001:153-174). A primitive stem cell, with great potential, gives rise to a proliferating progenitor pool, which in turn gives rise to recognizable differentiated cells. During this process, proliferative potential is lost, while specific differentiated features are acquired. Presumptively, but without definite proof, there is also self-renewal at the most primitive stem cell level, and this is also lost with differentiation. Many data exist to support such a hierarchical model. Marrow cells have been separated with short- and long-term repopulation potential (Guenechea, G. et al. (2001) Nat. Immunol. 2:75-82; Harrison, D.E. and Astle, C.M. (1997) Blood 90:174-181) and progenitors have been characterized as exclusively committed to the production of restricted progeny (Akashi, K. et al. (2000) Nature 404:193-197; Borge, O.J. et al. (1999) Blood 84:3781-3790). In addition, the clear expansion of different progenitor types in cytokine-stimulated in vitro culture with a loss of long-term engraftment capacity speaks to the existence of a progenitor hierarchy, at least at the more differentiated progenitor levels (McNiece, I.K. et al. (2002) Exp. Hematol. 30:612-616; Wiesmann, A. et al. (2000) Exp. Hematol. 28:128-139).

A model encompassing these features is described in Quesenberry, P.J. et al. ((2002) Blood 100:4266). However, this model does not fit with all published data. For instance, the "daughter cell" or paired-progenitor experiments of Suda and colleagues (Suda, T. et al. 1984) Proc. Natl. Acad. Sci. USA 81:2520-2524) indicate that a primitive progenitor cell can make totally different lineage choices during one cell cycle transit, such that, for example, one daughter cell forms an erythroid colony, while the other daughter (or sister) cell forms a neutrophil-macrophage colony. Out of a total of 387 pairs evaluated, 68 pairs of colonies consisted of dissimilar combinations of cell lineages. However, these studies were carried out using spleen as a cell source with erythropoietin and an uncharacterized conditioned media as a source of stimulation. In addition, others have reported high degrees of congruence in the phenotype of individual colonies observed after replating single cells from colony starts (Brady, G. and Iscove, N.N. (1993) Methods Enzymol. 225:611-623).

An instrinsic component of this hierarchical model is that the most primitive hematopoietic stem cell is a quiescent cell in G0 and a fount of potential without differentiated characteristics. It has generally been believed that primitive hematopoietic stem cells were dormant or quiescent and were thus protected from depletion or exhaustion (Hodgson, G.S. and Bradley, T.R. (1979) Nature 281:381-382; Lerner, C. and Harrison, D.E. (1990) Exp. Hematol. 18:114-118). On the basis of a number of transplant studies, a clonal succession model has been proposed (Kay, H.E.M. (1965) Lancet 2:418). This model proposes that the production of blood cells is maintained sequentially by one or just a few lymphohematopoietic stem cells, the residual stem cells remaining dormant. This model is consistent with reports evaluating the contributions of individually marked hematopoietic stem cells in transplant studies (Mintz, B. et al. (1984) Proc. Natl. Acad. Sci. USA 81:7835-7839; Lemischka, I.R. et al. (1986) Cell 45:917-927; Capel, B. et al. (1990) Blood 75:2267-2270). This scenario formed the basis for the concept that hematopoietic stem cells are hierarchically ordered based on their relative quiescence (Rosendaal, M. et al. (1979) Cell Tissue Kinet. 12:17-29; Hodgson, G.S. et al. (1982) Exp. Hematol 10:26-35).

In chemoradiotherapy and/or stem cell transplantation approaches, a major risk remains the cytopenic period occurring before stem cells have engrafted and produced differentiated progeny. The administration of more differentiated progenitor cells which might rapidly produce desired cell populations has intrigued investigators but has been limited by the number of available progenitor cells for such transplant support.

Accordingly, it would be desirable to have methods of producing differentiated progenitor cells for use in the treatment and/or prevention of cytopenia.

### SUMMARY OF THE INVENTION

The present invention generally relates to methods for the production of differentiated hematopoietic cells (e.g., megakaryocytes, platelets, and/or granulocytes) comprising culturing bone marrow stem cells under conditions that promote synchronous progression through the cell cycle, contacting the cells with at least one or more growth factors and/or cytokines (e.g., G-CSF, GM-CSF, and/or steel factor) at a predetermined phase of the cell cycle, and subculturing the cells until differentiated hematopoietic cells are produced (e.g., about 14 days). Preferably, synchronous progression through the cell cycle is induced by culturing the cells in the presence of steel factor, thrombopoietin, and FLT3-ligand.

In a preferred embodiment, the cells are contacted with G-CSF, GM-CSF, and steel factor at about mid-S phase, or about 32 hours after initiation of synchronous progression through the cell cycle. After about 14 days of culture, these cells differentiate into megakaryocytes which produce platelets, as well as proliferative granulocytes. In another preferred embodiment, the cells are contacted with G-CSF, GM-CSF, and steel factor at about late S phase, or about 40 hours after initiation of synchronous progression through the cell cycle. After about 14 days in culture, these cells differentiate into mature (non-proliferative) granulocytes.

In another preferred embodiment, the methods of the invention further comprise isolating the differentiated hematopoietic cells from undifferentiated or differently differentiated cells in the subculture.

In still another preferred embodiment, the invention provides differentiated hematopoietic cells produced according to the methods of the invention, as well as methods of treatment and/or prevention comprising administering the differentiated hematopoietic cells to subjects who have, or are at risk of developing, cytopenia.

Other aspects of the invention are disclosed -*infra.*

### BRIEF DESCRIPTION OF THE DRAWINGS

Figure 1 depicts a schematic of an exemplary (non-limiting) embodiment of the differentiation methods of the instant invention.

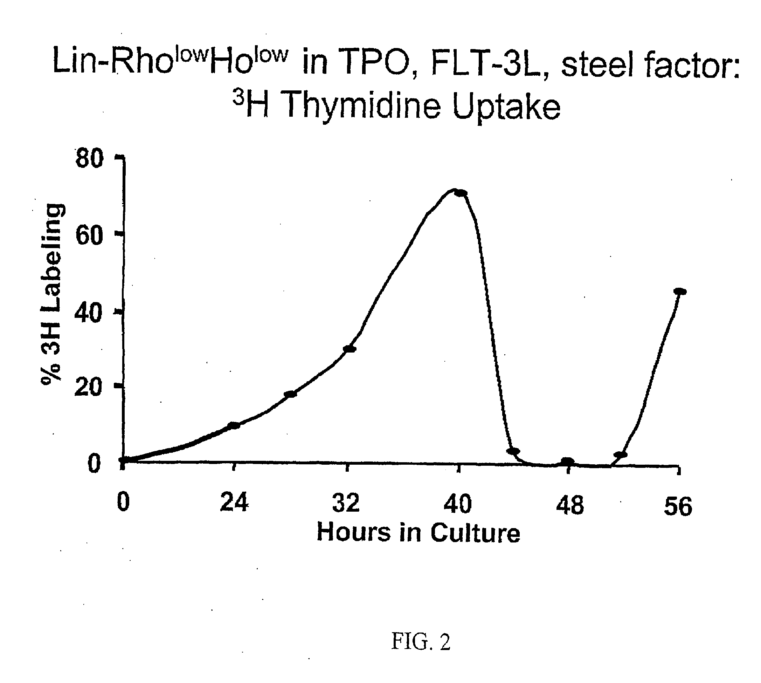

Figure 2 depicts a graph of the cell cycle phase over time, as determined by 3H incorporation, of synchronized murine stem cells in culture.
Figure 3 depicts a graph of proliferative granulocyte numbers after 14-day subculture of murine stem cells removed from initial culture at the indicated time points. A substantial increase in proliferative granulocytes is seen in cells subcultured after 32 hours of initial culture.
Figure 4 depicts a graph of non-proliferative granulocyte numbers after 14-day subculture of murine stem cells removed from initial culture at the indicated time points. A substantial increase in non-proliferative granulocytes is seen in cells subcultured after 40 hours of initial culture.
Figure 5 depicts a graph of megakaryocyte numbers after 14-day subculture of murine stem cells removed from initial culture at the indicated time points. A substantial increase in megakaryocytes is seen in cells subcultured after 32 hours of initial culture.
Figure 6 depicts a graph of the numbers high proliferative potential colony-forming cells (HPP-CFCs) the G₁ phase of the cell cycle.

### DETAILED DESCRIPTION OF THE INVENTION

The present invention is based, at least in part, on the discovery that purified bone marrow stem cells can be induced to differentiate in vitro into different hematopoietic cell types via cytokine stimulation at different points in the cell cycle. In one embodiment, stem cells can be induced to differentiate into megakaryocytes and/or platelets. In another embodiment, stem cells can be induced to differentiate into granulocytes. Accordingly, the present invention is useful in the treatment of cytopenic states characterized by thrombocytopenia and/or granulocytopenia (e.g., thrombocytopenia and/or granulocytopenia associated with cancer chemotherapy/chemoradiotherapy, bone marrow transplatation, and/or stem cell transplantation).

Previous work has shown shifts in murine marrow stem cell engraftment phenotype tied to cell cycle phase. These initial studies were carried out on both unseparated murine marrow and on highly purified LRH marrow stem cells. Critical aspects of these observations were that they occurred in the first cytokine induced cell cycle transit before cell division, and that they were reversible. There were also marked shifts observed in 7-factor responsive progenitors in the first cell cycle which were also reversible. Of great interest were observations linking an increase in progenitors to a decrease in engraftable stem cells; a so-called progenitor/stem cell inversion. These too were reversible. These latter observations were made on unseparated marrow cells.

The discoveries of the instant invention extended the above-described basic studies to specific phases of the cell cycle using purified murine bone marrow stem cells. Specifically, the discoveries of the instant invention establish the existence of differentiation "hotspots" at certain times in the cell cycle, that is, points where a specific differentiation pathway is favored. Accordingly, the differentiated hematopoietic cells of the invention are also referred to interchangeably herein as "hotspot cells" or "hotspot subcultured cells".

### I. Cell Culture Methods for bone marrow stem cells

The bone marrow stem cells used in the methods of the invention are preferably derived from a mammal, more preferably from a human. Preferably, the stem cells used in the method of the invention are from the same species to which the differentiated hematopoietic cells will be administered. For example, if differentiated hematopoietic cells are desired for administration to a human subject, then human stem cells will be used to produce the differentiated hematopoietic cells. If differentiated hematopoietic cells are desired for administration to a murine subject, then murine stem cells will be used to produce the differentiated hematopoietic cells. Even more preferably, the stem cells used in the methods of the invention are derived from the same subject to which the differentiated hematopoietic cells will be administered. The use of stem cells from the same subject removes the risk of a subsequent immune response between the subject and the differentiated hematopoietic cells.

Methods of isolating human and murine stem cells are known in the art. For example, murine LRH stem cells may be isolated according to the methods described below in the Examples- Human stem cells can be isolated based on the presence of the CD34 cell-surface antigen. Methods for the isolation of human CD34+ stem cells are described, for example, in U.S. Patent No. 5,635,387.

The growth factors and/or cytokines used to differentiate the cells during subculture may be any number and/or combination of growth factors and/or cytokines known in the art. In a preferred embodiment, the cells are cultured in the presence of G-CSF, GM-CSF, and steel factor (also referred to in the art as stem cell factor (SCF)). In other embodiments, the cells may also be cultured in the presence of growth factors and/or cytokines including, but not limited to, CSF-1, erythropoietin, FLT-3 ligand (FLT-3L), thrombopoietin (TPO), C-kit ligand, interleukins 1-26 (IL-1 to IL-26), FGF-1, PDGF, and EGF. It will be understood by those of skill in the art will appreciate that the methods of the invention include the use of any of the above-described growth factors and/or cytokines alone or in combination.

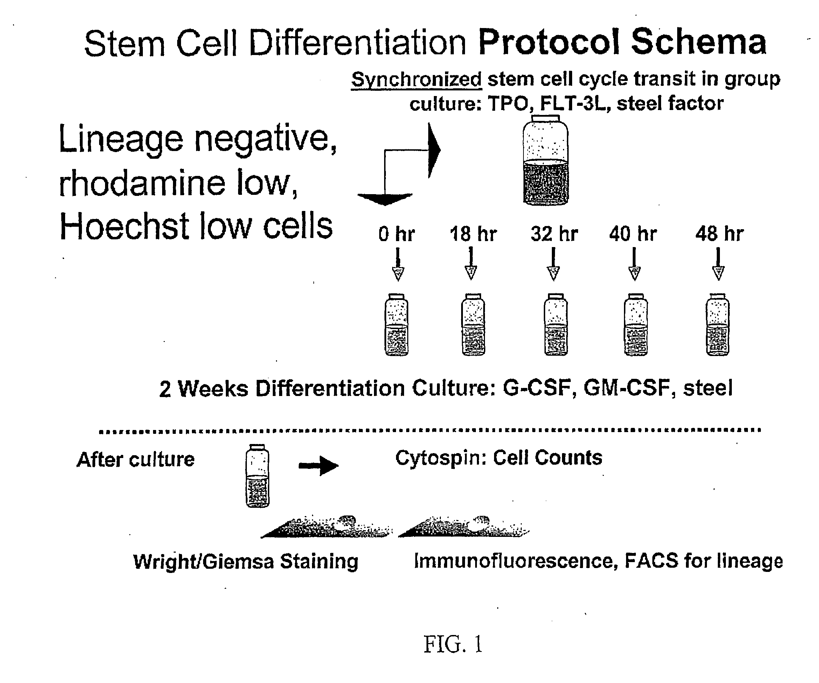

In an exemplary embodiment, the methods of the invention (when applied to murine cells) comprise the culture of unseparated or purified LRH marrow stem cells (or unseparated marrow). Specifically LRH cells are cultured in DMEM with 15% fetal calf sera and steel factor (50 ng/ml), Flt-3 (100 ng/ml), and thrombopoietin in Teflon bottle cultures at 37°C, 5% CO2. Under these conditions, primitive stem cells progress through cell cycle in a highly synchronous fashion. These cells are then harvested at about 32 hours (mid S-phase) or 40 hours (late S-phase), washed, and subcultured in DMEM, 15% fetal calf sera, and GM-CSF, G-CSF, and steel factor (50 ng/ml) and differentiated cell production evaluated out to 14 days of subculture. See Figure 1 for a schematic representation. When 32 hour primary cultured cells are resubcultured, there is a marked increase in megakaryocyte production, while when 40 hour primary cultured cells are resubcultured, a marked increase in granulocytes is seen. These primary time points are referred to herein as megakaryocyte and granulocyte "hotspots", respectively, and the differentiated hematopoietic cells resulting from these subcultures are referred to herein as "32 hour hotspot cells" and "40 hour hotspot cells", respectively.

### II. Methods of Treatment

The present invention includes methods of treatment comprising administration of the differentiated hematopoietic cells produced according to the methods described herein. The differentiated hematopoietic cells are particularly useful for treating subjects in which it is desirable to administer hematopoietic cells which can perform their respective functions in the subject rapidly, i.e., cells which are already differentiated at the time of administration, as opposed to stem cells, which take more time to differentiate after administration.

For example, the administration of 32 hour hotspot cells (as defined elsewhere herein) with high levels of megakaryocyte and platelet production can be used to acutely restore platelet levels in a subject (e.g., a human or mouse subject) having or at risk of developing a cytopenic condition or disorder including, but not limited to: thrombocytopenia associated with chemotherapy; thrombocytopenia associated with radiation therapy; aplastic anemia; thrombocytopenia associated with immune dysfunction; thrombocytopenia associated with disseminated intravascular coagulation; thrombocytopenia associated with thrombotoic thrombocytopenia purpura; and thrombocytopenia associated with collagen vascular diseases such as lupus erythematosus and scleroderma.

Administration of 32 hour hotspot cells may also be useful in the treatment of subjects with platelet-associated disorders. As used herein, a "platelet-associated disorder" includes a disorder, disease or condition which is caused, characterized by, related to, or associated with a decreased level of platelets. Platelet associated disorders, as used herein, also include disorders, diseases, or conditions which can be improved and/or treated by the administration of platelets. Platelet-associated disorders can detrimentally affect cellular functions such as blood-clotting, as well as other functions such as cellular proliferation, growth, differentiation, or migration, inter- or intra-cellular communication, tissue function, and systemic responses in an organism, such as immune responses. Preferred examples of platelet-associated disorders include, but are not limited to, immune disorders, septic shock, cancer (e.g., leukemias such as acute megakaryocytic leukemia, megakaryoblastic leukemia), infectious disease, stroke, heart disease, myocardial infarction, vascular disorders, arteriosclerosis, clotting and/or bleeding disorders, and platelet insufficiency.

Further examples of clotting and/or bleeding disorders include, but are not limited to, Hemophilia A (Factor VIII deficiency), Hemophilia B (Factor IX deficiency), von Willebrand disease, P-thalassemia, deep-vein thrombosis, thrombocytopenia, Immune Thrombocytopenic Purpura, Idiopathic Thrombocytopenic Purpura, Thrombotic Thrombocytopenic Purpura, hypercoagulation, hypocoagulation, protein S deficiency, protein C deficiency, Factor V Leiden, Factor XI deficiency (Rosenthal Syndrome or Plasma Thromboplastin Antecedent (PTA) deficiency), Factor XII deficiency (Hageman factor deficiency), Vitamin K deficiency, generalized coagulopathy, Factor XIII deficiency, Factor VII deficiency, internal bleeding, gastrointestinal bleeding, intracranial bleeding, Afibrinogenemia, Dysfibrinogenemia, Factor II disorders, Factor III (tissue factor) associated disorders, Factor V (labile factor) deficiency, Factor X deficiency, Factor V & VIII Combined Deficiency, Factor VIII & IX combined Deficiency, Factor IX & XI Combined Deficiency, Thrombophilia (Antithrombin III deficiency), Giant Platelet Syndrome (platelet glycoprotein Ib deficiency), Fletcher Factor Deficiency (Prekallikrein deficiency), Autosomal dominant macrothrombocytopenia, the May-Hegglin anomaly, Sebastian syndrome, Fechtner syndrome, platelet storage pool deficiency, Chediak-Higashi syndrome, amegakaryocytic thrombocytopenia, thrombocytopenia with absent radii (TAR), radioulnar stenosis, familial platelet disorder with predisposition to acute myelocytic leukemia (FPD-AML), and clotting and/or bleeding disorders or conditions associated with surgery, organ transplants, bone marrow transplants, chemotherapy, and/or other medical procedures and/or treatments.

As used herein a "platelet", also referred to as a "thrombocyte", are nucleus-free cytoplasmic fragments derived from large cells in the bone marrow, the megakaryocyte. The central portion of a platelet stains purple with Wright's stain and is referred to as the granulomere. The peripheral portion stains clear and is called the hyalomere. Normal platelet counts range from 150,000 to 400,000 per cu/ml blood. Platelets play a crucial part in the blood clotting process by forming a platelet plug. This is a two step process. First, single platelets bind to the site of the wound (adhesion). Next, the platelets bind to each other (activation). Activation can be stimulated by components released when the blood vessel is damaged and by thrombin, released during the blood clotting process. When platelets become activated they change. They release agents which recruit and activate the surrounding platelets. The result of these two processes is the formation of fibrin which stabilizes the platelet plug, stops bleeding and allows injuries to heal.

As used herein, a "platelet-mediated activity" includes an activity which involves the action of platelets. Platelet-mediated activities include adhesion to the site of a wound, activation (e.g., release of blood clotting factors), induction of blood clotting (e.g., induction of fibrin formation), inhibition of bleeding, and induction of wound healing.

In another embodiment, the administration of 32 or 40 hour hotspot cells (as defined elsewhere herein) with high levels of granulocyte production can be used to acutely restore granulocyte levels in a subject (e.g., a human or mouse subject) having or at risk of developing a cytopenic condition or disorder including, but not limited to: granulocytopenia associated with chemotherapy; granulocytopenia associated with radiation therapy; congenital granulocytopenia.

In general, methods for bone marrow/stem cell transplants after myeloablation with irradiation or chemotherapy involve infusion of marrow cells intravenously after the specific ablative therapy. In murine animals, the in vivo quantitation of marrow renewal of stem cells may be accomplished by competing "marked" donor cells (e.g., CD45.1 or male) with distinguishable competitor cells (e.g., CD45.2 or female) into lethally irradiated CD45.2 or female recipient hosts. Engraftment is then determined at various times after cell infusion by determining the percentage of CD45.1 or male marrow or blood cells. For short term renewal transplant studies, cultured "hotspot" cells are infused into lethally irradiated mice and recovery of platelets or granulocytes evaluated.

As used herein, "treatment" of a subject includes the application or administration of a therapeutic agent (e.g., differentiated hematopoietic cells) to a subject, e.g., a subject suffering from or at risk for developing cytopenia (e.g., thrombocytopenia and/or granulocytopenia), with the purpose of curing, healing, alleviating, relieving, altering, remedying, ameliorating, improving, preventing or affecting the cytopenia and the symptoms associate therewith. It will be understood by those of skill in the art that a "method of treatment" does not require a complete "cure", or a complete absence of symptoms resulting from the cytopenic state. A "method of treatment" refers to any method wherein the performance of the method leads to a beneficial effect in the subject on whom the method is performed.

The term "therapeutically effective amount" refers to a sufficient amount of differentiated hematopoietic cells (e.g., megakaryocytes, platelets, and/or granulocytes) delivered to produce an adequate therapeutic response in a subject, e.g., prevention or treatment of cytopenic symptoms.

The present invention is further illustrated by the following examples- These examples are provided to aid in the understanding of the invention and are not construed as a limitation thereof.

### EXAMPLES

### EXAMPLE 1: DIFFERENTIATION STUDIES OF PURIFIED PROGENITOR [LINEAGE^{NEGATIVE}RHODAMINE^{LOW}HOESCHT^{LOW} (LRH) CELLS.

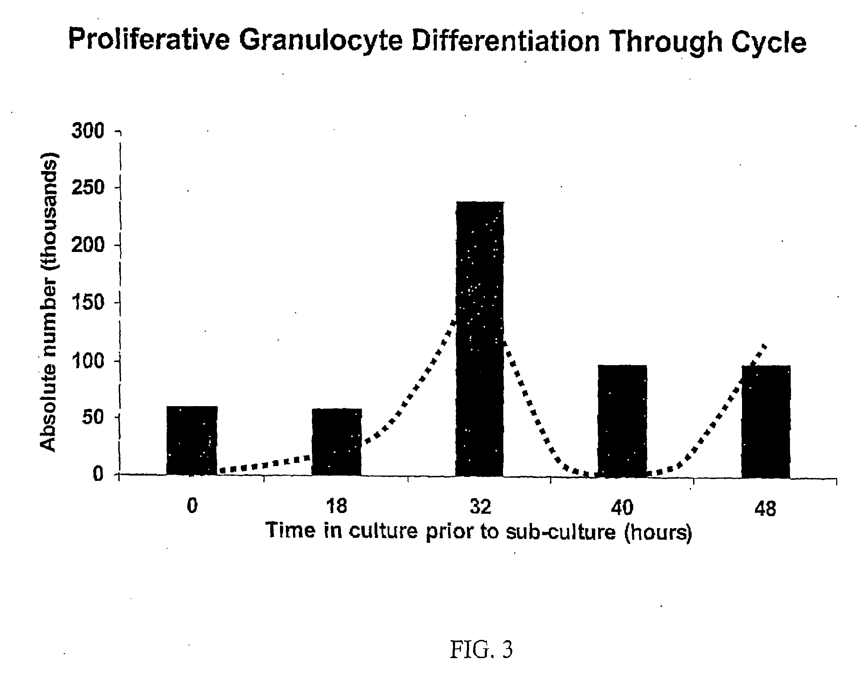

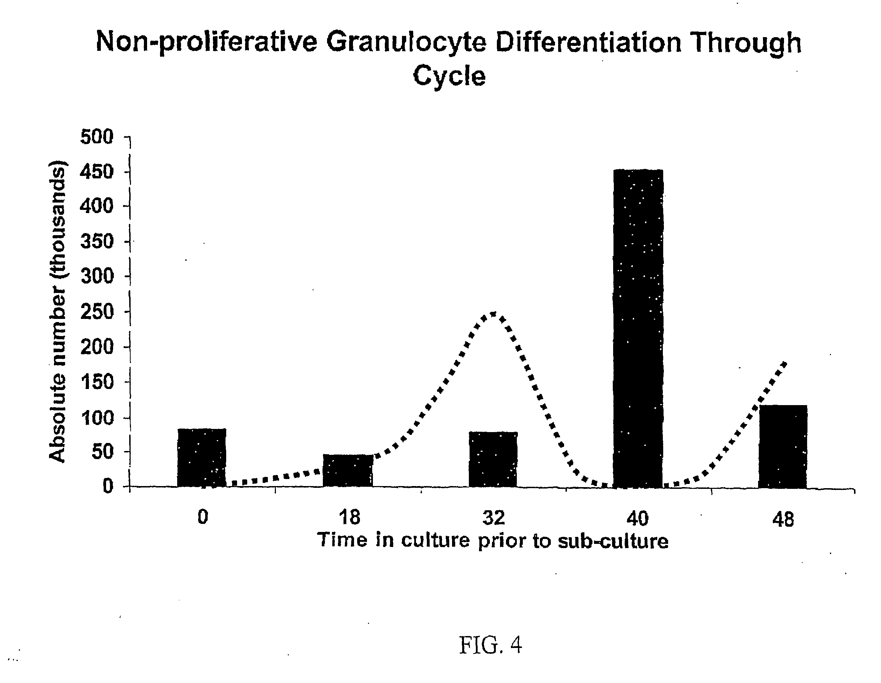

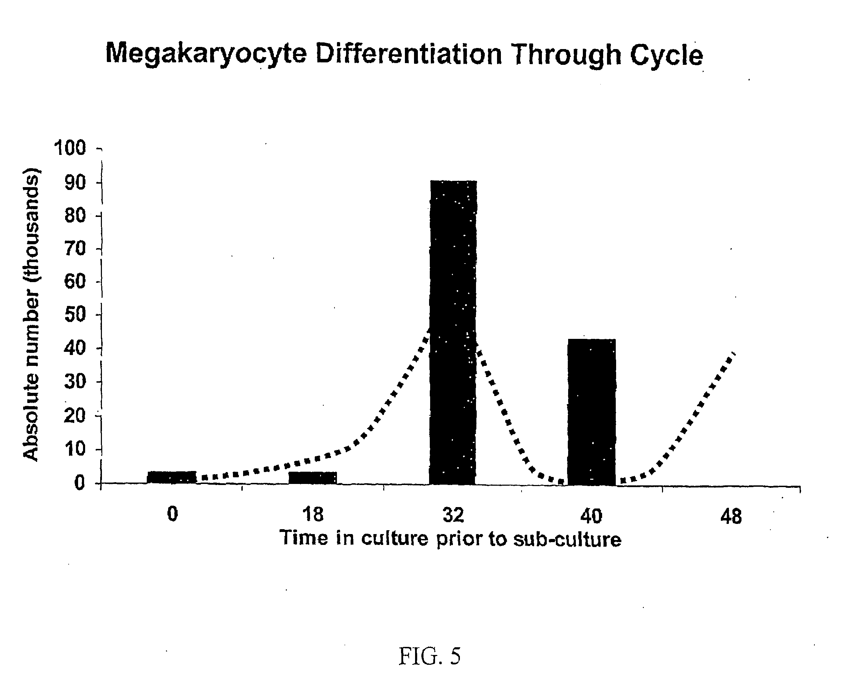

LRH cells were primarily cultured in FLT3-ligand, thrombopoietin, and steel-factor (FTS) for cycle initiation from resting Go state and then serially sub-cultured (1,000 cells/vessel) in inductive differentiation cocktails prior to cell division- GM-CSF and G-CSF were used together at three different log-dilutions; steel-factor was present in all cultures at 50 ng/ml. See Figure 1 for a schematic representation of the culture methods. Marked variations in differentiated cell production were seen with the first cell cycle transit. Megakaryocyte differentiation was amplified from 3.5x10³ cells at Go to 9.1x10⁴ cells in mid-S phase (Figure 5)- Proliferative granulocyte differentiation was amplified from 6.0x10⁴ cells at Go to 2.4x10⁵ cells in mid-S phase (Figure 3. Non-proliferating granulocyte differentiation was amplified from 8.2x10⁴ cells at Go to 4.6x10⁵ cells in late-S phase (Figure 4). These data further support a flexible system for hematopoietic regulation in which multiple different outcomes could occur dependent on cell cycle phase and specific microenvironmental influences. Early primitive marrow stem/progenitor cells represent a continuum of reversible phenotypic shifts as opposed to a hierarchy, with continuous change in a reversible fashion.

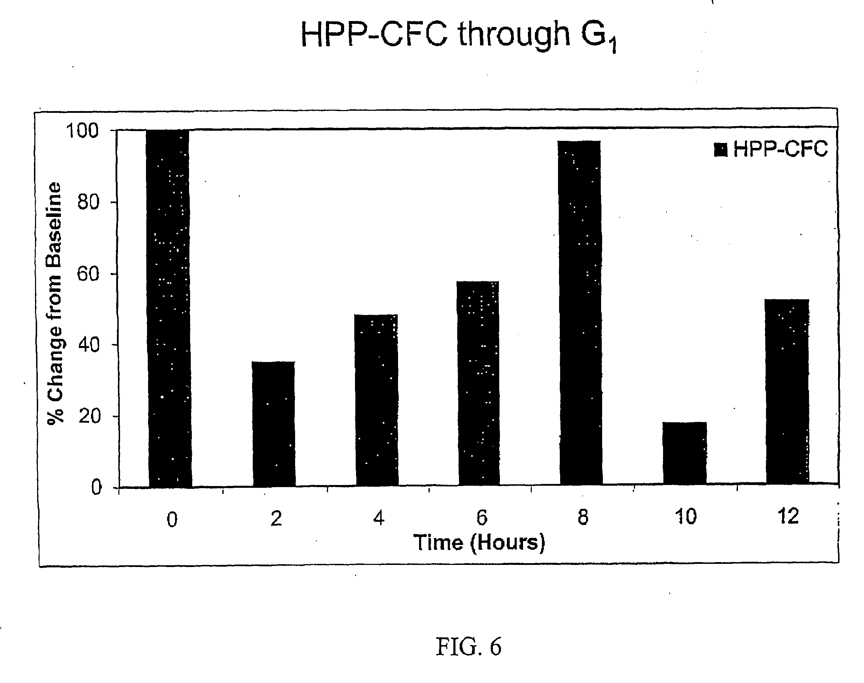

B6.SJL marrow cultured in FTS showed marked fluctuations in total HPP-CFC during the G₁ phase of the cell cycle. HPP-CFC was at 35% of input at 2-hours and returned to 96% of input values after 8-hours of cytokine-culture. There was a second nadir (17% of input) at 10-hours. See Figure 6.

### Materials and Methods:

### Marrow Cultures

Bone marrow was flushed from the femurs, tibiae and iliac crests of 6-8 week old mice with Hank's Balanced Salt Solution (HBSS, Life Technologies, Gibco/BRL, Rockville, MD) using 22-guage needles. The marrow cells were cultured at a density of 1-5x10⁶ cells/ml in Dulbecco's Modified Essential Medium low glucose (Life Technologies, Gibco/BRL) containing 15% heat-inactivated fetal calf serum (HyClone Laboratories, Logan, UT), 1% penicillin (100 U/ml), 1% streptomycin (100 µg/ml) and 1% L-glutamine (100 µg/ml). Cell numbers were determined using a hemocytometer with 0.01 % (W/V) solution of crystal violet in water containing 2% (V/V) acetic acid. The cytokine cocktail utilized combined recombinant murine (rm) FLT-3L (100 ng/ml; R & D), thrombopoietin (200 ng/ml), and rm steel factor (50 ng/ml; R & D). The bone marrow was cultured with the above cytokines for various times in nonadjacent Teflon bottles or in slow turning later vessels at 10 rpm in a humidified 5% CO₂, 3°C water-jacketed incubator (Forma Scientific Inc., Marietta, OH).

### Isolation of lineage negative, rhodamine^{low}Hoechst 33342^{low} (LRH) purified stem cells

Bone marrow was isolated from the iliac bones, femurs, and tibiae of male BALB/c or B6.SJL mice of 6-8 weeks of age. A low-density fraction (<1.077 g/cm²) was isolated on Nycoprep 1.044A (Accurate Chemical and Scientific Corporation, Westbury, NY). The cells were lineage depleted with the following antibodies: TER 119, B220, Mac-1, GR-1, Lyt-2, L3T4, YW25.12.7, and Dynabeads M450 anti-rat IgG (Dynal, Lake Success, NY) (Boswell et al. (1984)). The lineage-depleted cells were labeled with rhodamine-123 at 0.1 µg/ml and Hoechst-3332 at 10 µM. The cells were incubated in the dark for 30 minutes at 37°C, allowing time for efflux of rhodamine from the marrow progenitors. The last incubation was carried out twice before sorting. A population with both low expression ofHoechst and rhodamine fluorescence was isolated by FACS using a high-speed multi-laster MoFlo® cell sorter (Cytomation, Fort Collins, Colorado) (Bertoncello et al. (1985); Baines et al. (1983)).

### Rotating Wall Vessel (RWV) Cultures

The rotary Cell Culture System^{™} (Synthecon Inc., Houston, TX) consists of a rotator base, power supply, and a rotating vessel. The RWV is a horizontally rotated, gas-free (zero head-space), transparent cylindrical culture chamber with a center core of a thin silicon rubber diffusion membrane for gas exchange. A reusable 55 ml slow turning later vessel was used for the experiments. The incubator air-mixture is drawn through an air pump into a 0.22 µm filter to diffuse through the cell culture at approximately 1 L/min. The assembled RWV is autoclaved for 30 minutes at 120°C before each use. Air bubbles that are present after filling the vessel were removed at 2, 4, 16, and 24 hours as needed after initiation of the culture when there was bubble coalescence. All residual air was successfully withdrawn by 24 hours of culture. Replacement of the air bubbles with cytokine-supplemented media was accomplished by placing syringes on the screws, one filled with media, the other empty. The residual air bubbles were teased out of the device with amalgamation in the empty syringe by positional change and tapping of the RWV in a sterile hood at the time of media infusion.

### Progenitor assays - High Proliferative potential colony forming cell (HPP-CFC) and colony forming unit-culture

Cultured cells under various conditions were assessed for in vitro colony forming unit culture (CFU-c) and high proliferative potential colony-forming cell (HPP-CFC) assay using a double layers of agar nutrient medium technique in 35 mm plastic culture dishes (Falcon). The bottom layer consisted of: 1.0 ml underlay of 0.5% agar (Difco, Detroit, MI) with α-MEM (Life Technologies/GibcoBRL), 20% fetal calf serum (Hyclone), 1% penicillin/streptomycin (Life Technologies/GibcoBRL) and 1% L-glutamine (Life Technologies/Gibco/BRL) and seven separate growth factors. These growth factors were: recombinant murine (rm) colony-stimulating factor-1 (CSF-1), 7,500 units per plate (R&D Systems, Minneapolis, MN), murine granulocyte-macrophage colony stimulating factor (GM-CSF), 3.75 ng per plate (R&D), murine interleukin (IL)-1α, 375 units per plate (R&D), rm IL-3, 150 units per plate (Collaborative Research, Bedford, MA), rm steel factor, 150 ng per plate (R&D), murine granulocyte colony stimulating factor (G-CSF), 7.5 ng per plate (R&D), and rm basic fibroblast growth factor (bFGF), 7.5 ng per plate (Collaborative). Cells were plated at a concentration of 20,000 cells per plate for the starting population, and after various times in culture, a culture volume equivalent to the starting volume which contained 20,000 cells was plated in a 0.5 ml overlayer of 0.3% agar in α-MEM with 20% heat-inactivated fetal calf serum (HI-FCS). Five culture dishes were set up per group per time point. The plates were incubated in a humidified 5% O₂, 10% CO₂ 85% N₂ 37°C water-jacketed incubator (Formal Scientific, Marietta, OH). After 2 weeks, the colonies were scored using a dissecting microscope at 20X magnification. dishes were scored for HPP-CFC and CFU-c. HPP-CFC were considered highly dense colonies over 0.5 mm in diameter or moderately dense colonies of over 1.0 mm. CFU-c were considered to be all other colonies of over 50 cells and not meeting the criteria for HPP-CFC. Total progenitors represented the combined values for HPP-CFC and CFU-c (Bertoncello, I. and Kriegler, A.B. (1997) Methods Mol. Biol. 75:265-272; Bertoncello, I. (1992) Curr. Top. Microbiol. Immunol. 177:83-94; Kriegler, A.B. et al. (1994) Exp. Hematol. 22:432-440; Lowry, P.A. et al. (1991) Exp. Hematol. 19:994-996; McNiece, I.K. et al. (1990) Int. J. Cell Cloning 8:146-160).

Those skilled in the art will recognize, or be able to ascertain using no more than routine experimentation, many equivalents to the specific embodiments of the invention described herein. Such equivalents are intended to be encompassed by the following claims.

## Claims

1. A method for the production of differentiated hematopoietic cells comprising:
a) culturing bone marrow stem cells under conditions that promote synchronous progression through the cell cycle;
b) contacting the cells with at least one growth factor and/or cytokine in mid S-phase or late S-phase; and
c) subculturing the cells until differentiated hematopoietic cells are produced.

2. The method of claim 1, wherein the at least one growth factor and/or cytokine is selected from G-CSF, GM-CSF, and/or steel factor.

3. The method of any one of claims 1-2, wherein culturing the cells under conditions that promote synchronous progression through the cell cycle comprises culturing the cells in the presence of steel factor, thrombopoietin, and FLT3-ligand.

4. The method of any one of claims 1-3, wherein the step of subculturing the cells is carried out for 14 days.

5. The method of claim 4, wherein mid-S phase occurs 32 hours after initiation of the culturing of the stem cells under conditions that promote synchronous progression through the cell cycle.

6. The method of any one of claims 1-5, wherein the differentiated hematopoietic cells comprise megakaryocytes.

7. The method of any one of claims 1-5, wherein the differentiated hematopoietic cells comprise platelets.

8. The method of any one of claims 1-5, wherein the differentiated hematopoietic cells comprise proliferative granulocytes.

9. The method of claim 1, wherein late S phase occurs 40 hours after initiation of the culturing of the stem cells under conditions that promote synchronous progression through the cell cycle.

10. The method of any one of claims 1 or 9, wherein step b involves contacting the cells with at least one growth factor and/or cytokine in late S-phase and wherein the differentiated hematopoietic cells comprise mature granulocytes.

11. The method of any one of claims 1-10, further comprising isolating the differentiated hematopoietic cells from the subculture.

## Patentansprüche

1. Verfahren zur Herstellung von differenzierten hämatopoetischen Zellen, das Folgendes aufweist:
a) Kultivieren von Knochenmarkstammzellen unter Bedingungen, die ein synchrones Fortschreiten durch den Zellzyklus fördern;
b) In-Kontakt-Bringen der Zellen mit zumindest einem Wachstumsfaktor und/oder Cytokin in der mittleren S-Phase oder späten S-Phase, und
c) Subkultivieren der Zellen, bis differenzierte hämotopoetische Zellen hergestellt sind.

2. Verfahren nach Anspruch 1, wobei der zumindest eine Wachstumsfaktor und/oder das zumindest eine Cytokin ausgewählt ist aus G-CSF, GM-CSF und/oder Stammzellfaktor.

3. Verfahren nach einem der Ansprüche 1 bis 2, wobei das Kultivieren der Zellen unter Bedingungen, die das synchrone Fortschreiten durch den Zellzyklus fördern, das Kultivieren der Zellen in Gegenwart von Stammzellfaktor, Thrombopoietin und FLT3-Ligand aufweist.

4. Verfahren nach einem der Ansprüche 1 bis 3, wobei der Schritt des Subkultivierens der Zellen für 14 Tage durchgeführt wird.

5. Verfahren nach Anspruch 4, wobei die mittlere S-Phase 32 Stunden nach der Initiation des Kultivierens der Stammzellen unter Bedingungen, die ein synchrones Fortschreiten durch den Zellzyklus fördern, stattfindet.

6. Verfahren nach einem der Ansprüche 1 bis 5, wobei die differenzierten hämatopoetischen Zellen Megakariocyten aufweisen.

7. Verfahren nach einem der Ansprüche 1 bis 5, wobei die differenzierten hämatopoetischen Zellen Plättchen aufweisen.

8. Verfahren nach einem der Ansprüche 1 bis 5, wobei die differenzierten hämatopoetischen Zellen proliferative Granulocyten aufweisen.

9. Verfahren nach Anspruch 1, wobei die späte S-Phase 40 Stunden nach der Initiation der Kultivierung der Stammzellen unter Bedingungen, die das synchrone Fortschreiten durch den Zellzyklus fördern, stattfindet.

10. Verfahren nach einem der Ansprüche 1, 4 oder 9, wobei Schritt b das In-Kontakt-Bringen der Zellen mit zumindest einem Wachstumsfaktor und/oder Cytokin in der späten S-Phase involviert, und wobei die differenzierten hämatopoetischen Zellen reife Granulocyten aufweisen.

11. Verfahren nach einem der Ansprüche 1 bis 10, das ferner das Isolieren der differenzierten hämatopoetischen Zellen aus der Subkultur aufweist.

## Revendications

1. Procédé de production de cellules hématopoïétiques différenciées comprenant :
a) la culture de cellules souches de moelle osseuse dans des conditions favorisant un développement synchrone au cours du cycle cellulaire ;
b) la mise en contact des cellules avec au moins un facteur de croissance et/ou une cytokine en phase S intermédiaire ou en phase S tardive ; et
c) la sous-culture des cellules jusqu'à ce que des cellules hématopoïétiques différenciées soient produites.

2. Procédé selon la revendication 1, dans lequel le moins un facteur de croissance et/ou cytokine est choisi parmi G-CSF, GM-CSF, et/ou le facteur Steel.

3. Procédé selon l'une quelconque des revendications 1 et 2, dans lequel la culture des cellules dans des conditions favorisant un développement synchrone au cours du cycle cellulaire comprend la culture des cellules en présence de facteur Steel, de thrombopoïétine et de ligand de FLT3.

4. Procédé selon l'une quelconque des revendications 1 à 3, dans lequel l'étape de sous-culture des cellules est effectuée pendant 14 jours.

5. Procédé selon la revendication 4, dans lequel la phase S intermédiaire intervient 32 heures après le début de la culture des cellules souches dans des conditions favorisant un développement synchrone au cours du cycle cellulaire.

6. Procédé selon l'une quelconque des revendications 1 à 5, dans lequel les cellules hématopoïétiques différenciées comprennent des mégacaryocytes.

7. Procédé selon l'une quelconque des revendications 1 à 5, dans lequel les cellules hématopoïétiques différenciées comprennent des plaquettes.

8. Procédé selon l'une quelconque des revendications 1 à 5, dans lequel les cellules hématopoïétiques différenciées comprennent des granulocytes proliférants.

9. Procédé selon la revendication 1, dans lequel la phase S tardive intervient 40 heures après le début de la culture des cellules souches dans des conditions favorisant un développement synchrone au cours du cycle cellulaire.

10. Procédé selon l'une quelconque des revendications 1, 4 ou 9, dans lequel l'étape b comporte la mise en contact des cellules avec au moins un facteur de croissance et/ou une cytokine en phase S tardive et dans lequel les cellules hématopoïétiques différenciées comprennent des granulocytes matures.

11. Procédé selon l'une quelconque des revendications 1 à 10, comprenant en outre l'isolement des cellules hématopoïétiques différenciées à partir de la sous-culture.
